# EUROPÄISCHE PATENTSCHRIFT

(11) **EP 1 385 492 B1**
(45) Veröffentlichungstag und Bekanntmachung des Hinweises auf die Patenterteilung: **21.03.2007**
(21) Anmeldenummer: 01987663.0
(22) Anmeldetag: 19.10.2001
(51) Int. Cl.: A61K 31/00, A61P 19/02

(54) **VERWENDUNG VON HYALURONSÄURE-DERIVATEN ZUR HEMMUNG VON ENTZÜNDLICHEN ARTHRITIDEN**
USE OF HYALURONIC ACID DERIVATIVES FOR THE PREVENTION OF INFLAMMATORY ARTHRITIS
UTILISATION DE DERIVES D'ACIDE HYALURONIQUE POUR INHIBER LES ARTHRITES INFLAMMATOIRES

(30) Priorität: 19.10.2000 DE 10053053
(43) Veröffentlichungstag der Anmeldung: 04.02.2004
(73) Patentinhaber: WPMO GmbH, 6304 Zug (CH)
(72) Erfinder: VENBROCKS, Rudolf, 07607 Hainspitz (DE); ROTH, Andreas, 07607 Eisenberg (DE); MÜLLER, Peter-Jürgen, 07745 Jena (DE); MÖLLER, Stephanie, 07749 Jena (DE); OZEGOWSKI, Jörg;, 07747 Jena; (DE); PESCHEL, Gundela, 07745 Jena (DE)
(74) Vertreter: Meyers, Hans-Wilhelm
(86) Internationale Anmeldenummer: PCT/DE2001/003984
(87) Internationale Veröffentlichungsnummer: WO 2002/032407

(56) Entgegenhaltungen:
- EP-A- 0 754 460
- WO-A-00/56298
- WO-A-88/07060
- WO-A-92/13541
- WO-A-95/25751
- WO-A-98/45335
- US-A- 4 801 619
- US-A- 4 808 576
- US-A- 5 079 236
- US-A- 5 409 904
- DATABASE CA [Online] CHEMICAL ABSTRACTS SERVICE, COLUMBUS, OHIO, US; BARBUCCI, ROLANDO ET AL: "Hyaluronan derivatives: chemical modifications and biochemical applications" retrieved from STN, accession no. 2000:449963 Database accession no. 134:9278 XP002253810 & INTERNATIONAL CONGRESS SERIES (2000), 1196(NEW FRONTIERS IN MEDICAL SCIENCES: REDEFINING HYALURONAN), 203-212 ,
- DATABASE CA [Online] CHEMICAL ABSTRACTS SERVICE, COLUMBUS, OHIO, US; UMEMOTO, JUNJI ET AL: "Medicinal compositions for inhibiting kallikrein-kinin system or phospholipase A2" retrieved from STN Database accession no. 134:518 XP002253811 -& DATABASE WPI Week 200105 Derwent Publications Ltd., London, GB; AN 2001-041010 XP002253812 & WO 200 069 917 A & WO 2000 069917 A (MARUHO KABUSHIKIKAISHA, JAPAN) 23. November 2000 (2000-11-23)
- DATABASE WPI Week 199953 Derwent Publications Ltd., London, GB; AN 1999-615548 XP002253813 & JP 11 269077 A (MARUHO KK) 5. Oktober 1999 (1999-10-05)
- GOODSHIP A E ET AL: "TREATMENT OF TENDONITIS IN HORSES" VETERINARY RECORD, BRITISH VETERINARY ASSOCIATION, LONDON, GB, Bd. 130, Nr. 3, 18. Januar 1992 (1992-01-18), Seite 58 XP008018663 ISSN: 0042-4900
- STOEVE J ET AL: "UEBERSICHT UEBER DIE KLINISCHE UND EXPERIMENTELLE ANWENDUNG DER HYALURONSAEURE BEI GONARTHROSE THE CLINICAL AND EXPERIMENTAL USE OF HYALURONIC ACID IN THE THERAPY OF GONARTHROSIS" ZEITSCHRIFT FUER ORTHOPAEDIE UND IHRE GRENZGEBIETE, ENKE, STUTTGART, DE, Bd. 137, Nr. 5, 1999, Seiten 393-399, XP008018820 ISSN: 0044-3220
- ADAM M: "ZUR INTRAARTIKULAEREN BEHANDLUNG MIT GLYCOSAMINOGLYKANPOLYSULFAT BEI CHRONISCHER POLYARTHRITIS" THERAPIEWOCHE, KARLSRUHE, DE, Bd. 35, Nr. 21, 1985, Seiten 2601-2602,2604,2606, XP008018847 ISSN: 0040-5973

## Beschreibung

Die Erfindung betrifft die Verwendung von neuen Formulierungen zur Herstellung eines Arzneimittels zur Behandlung von Rheumatoid-Arthritis (RA). RA ist eine chronisch entzündliche Erkrankung, welche über mehrere Stadien zuletzt zur massiven Gelenkdestruktion oder auch zu Entzündungen im Bereich der Sehnen führt. Man geht nach dem jetzigen Erkenntnisstand davon aus, dass T-Zellen die Entzündung initiieren und aufrechterhalten. In diesem Prozess wirken Zytokine sowie mesenchymale Zellen (Makrophagen und synoviale Fibroblasten). Ein Entzündungsmediator ist dabei sehr wahrscheinlich das Zytokin TNF-a. Dieses Zytokin wird hauptsächlich durch die Makrophagen freigesetzt. Es unterstützt die Bildung des für die RA typischen Pannus, welcher die Knorpeldestruktion fördert. TNF-α erhöht die Zahl der Adhäsionsmoleküle für Leukozyten auf der Oberfläche der Endothelzellen und die Fenestrierung der kapillären Endothelschicht. Dies führt zu einem vermehrten Einstrom von Leukozyten in die Synovia. Das Zytokin fördert die Sekretion von Matrix-Metalloproteinasen durch die Synoviozyten. Diese Enzyme sind direkt an der Zerstörung von Knochen und Knorpel beteiligt. TNF-a sensibilisiert auch Schmerzrezeptoren, verbunden mit der Induktion von Schmerzgefühlen. TNF-a hat eine entscheidende Rolle bei der Initiierung und Unterhaltung der rheumatischen Arthritis.

Hyaluronsäure wird sowohl in Tieren als Bestandteil der Synovialflüssigkeit der Gelenke und anderer Gewebe z. B. des Glaskörpers des Auges, und wird auch von Bakterien der Gattung *Streptococcus* gebildet. Durch ihre viskoelastischen Eigenschaften erhöht sie die Gleitfähigkeit der Gelenke und wirkt als Stoßfänger. Sie bildet viscoelastische Lösungen.
Hyaluronsäure ist ein körpereigenes Glucosaminoglycan, das in wiederholter Reihenfolge ein Disaccharid bestehend aus D-Glucuronsäure und N-acetyl-D-glucosamin enthält. Jedes Disaccharid ist über eine β-(1-4)-Bindung mit dem nächsten verbunden. Diese Bindung kann durch das ebenfalls körpereigene Enzym Hyaluronidase hydrolytisch gespalten werden. Es existiert ein Gleichgewicht Auf- und Abbau (turnover) der Hyaluronsäure im Körper. Unter dem Einfluß von Entzündungen verursachenden Radikalen wird die Hyaluronsäure sukzessiv abgebaut, wobei ihre viscoelastischen Eigenschaften abnehmen. Im Verlauf der RA wird demnach die Gleichgewichtslage des tumover der körpereigenen Hyaluronsäure gestört Uronide der Hyaluronsäure werden durch Einwirkung des Enzyms Hyaluronatlyase auf Hyaluronsäure hergestellt (WO 0038647).

Nach dem Stand der Technik werden Arzneimittel mit symptommodifizierender Wirkung und Substanzen mit chondroprotektiver bzw. strukturmodifizierender Wirkung unterschieden. Zur medikamentösen Behandlungen der RA werden eine große Zahl oral und subcutan verabreichter steroidaler und nicht-steroidaler Antirheumatika und eine Gruppe von Medikamenten, die eine hohe Ähnlichkeit mit der Synovialflüssigkeit oder Aufbaustoffen des Knorpels haben und intraarticulär (i.art.) verabreicht werden, eingesetzt. Die steroidalen Antirheumatika wirken systemisch als Entzündungshemmer.
Oral verabreicht wird z.B. Methotrexat (Lantarel), ein Purinantagonist und Zytostatikum und Leflunomid (Arava), ein Pyrimidin-Antagonist und Immunsuppresivum. Subcutan verabreicht wird z.B. Etanercept (Enbrel), ein TNF-α-Hemmer. Andere Pharmaka sind Hydroxychloroquinsulfat, Gold (Tauredon), und Penicillamin (Trisorcin).
Caruso et al. (US 4.312.866) beschreibt eine Therapie der Behandlung von RA mit dem Antibiotikum Rifamycin SV in Kombination mit basischen Aminosäuren.

Chondroprotektiva sind z.B. Gemische von Mucopolysacchridpolyschwefelsäureestern tierischer Herkunft, die sowohl bei der degenerativen Arthrose als auch bei der RA eingesetzt werden können. Ein polysulfatiertes Glycosaminoglycan ist unter der Bezeichnung Arteparon oder Adequan RTM für den Einsatz bei Tieren (Luitpold Pharmaceuticals) mit Molmassen zwischen 3.000 und 17.000 D im Einsatz. Die polysulfatierten Glycosaminoglycane fördern die Bildung von Hyaluronsäure an der synovialen Membran (Nishikawa et al. in" Influence of sulfated glycosaminoglycans on biosynthesis of hyaluronic acid in rabbit knee synovial membran"; Arch.

Biochem. Biophys., 240, 146 - 153). Das monomere Glucosaminsulfat (Dona, Rottapharm) wird auch als Mittel zur Behandlung der Arthrose eingesetzt.
Sulfatierte Hyaluronsäure oder sulfatiertes Dextran besitzen entzündungshemmende, systemisch wirkende Eigenschaften (JP 8.277.224). Es wird vorgeschlagen, sie in Mengen von 0,1 bis 10 mg/kg Körpergewicht intravenös z.B. bei Atemnotzustände in Form einer Lösung, welche 0,5 bis 10 mg/ml sulfatierte Polymer enthält, zu injizieren.
Sulfatierte Hyaluronsäure ist weiterhin bekannt für ihre dem Heparin ähnlichen anticoagulanten und antithrombolytischen und entzündungshemmenden Eigenschaften. Außerdem werden die Zelladhäsion verringernde Effekte beschrieben.
Lanfranco et al. (WO 9.845.335 und WO 9.943.728) setzen sulfatierte Hyaluronsäure in pharmazeutischen Formulierungen und Biomaterialien und für die Beschichtung von biomedizinischen Objekten ein.
Cialdi et al. (US 6.027.741) beschreiben sulfatierte Polysaccharide, z.B. sulfatierte Hyaluronsäure und deren Ester als anticoagulante und die Zelladhäsion reduzierende Eigenschaften für den Einsatz in Biomaterialien.

Die reine Hyaluronsäure, die eine gute Wirkung (US 4.808.576) bei degenerativer Arthrose entfaltet, gehört zu den langsam agierenden Medikamenten, mit einer Wirkung erst nach 3-5 Injektionen, wobei ein chondroprotektiver Effekt beobachtet werden kann. Dieser Effekt ist bei Therapien der RA bisher nicht nachgewiesen worden, so dass unmodifizierte Hyaluronsäure bei RA kaum eingesetzt wird. Die handelsüblichen Injektionspräparate mit Hyaluronsäure mit einem mittleren Molekulargewicht zwischen 500.000 D und 900.000 D besitzen im Krankheitsbild der RA somit keine Wirkung.

Eine praktizierte Methode der Behandlung der RA ist die i.art. Injektion von Spritzpräparaten mit Hyaluronsäure (Lindblad, US 4.801.619), welche Zusätze mit entzündungshemmenden Eigenschaften enthalten. Diese Zusätze sind zum Beispiel Steroide wie Prednisolon, Dexamethason aber auch Ibuprofen (Antirheumatikum) oder sulfatierte Mucopolysacchride als Nebenprodukt in der aus tierischem Material hergestellten Hyaluronsäure (Drizen et al. US 5.079.236). Ein Nachteil der vorgeschlagenen sterilen Formulierungen für die veterinärmedizinische Anwendung bei US 5.079.236 ist, dass die Sterilität durch den Zusatz von Konservierungsstoffen wie den zelltoxischen Parabenen erreicht wird. Die angegebenen Mengen von sulfatierten Mucopolysacchariden betragen zwischen 0,75 bis 1,25 %, bezogen auf die angegebenen 5 bis 20 mg Natriumhyaluronat pro ml Injektionslösung, d.h. es sind zwischen 0,037 und 0,25 mg/ml sulfatierte Mucopolysaccharide in dem Präparat als Verunreinigung enthalten.

Nach dem Stand der einschlägigen Technik besteht ein Mangel an topisch anzuwendenden, spezifisch gegen die unterschiedlichen Entwicklungsstadien und Ausprägungen des Krankheitsbildes der Rheumatoid-Arthritis wirkenden Arzneimittel.
Es ist das Ziel der vorliegenden Erfindung, solche spezifisch wirksame therapeutische, topisch wirkende Formulierungen zur gezielten Behandlung von Rheumatoid-Arthritis vorzuschlagen. Aufgabengemäß sollen die Formulierungen gut verträglich sein, eine nichttierische Herkunft besitzen und eine möglichst definierte chemische Zusammensetzung aufweisen.

Die Erfindung betrifft die Verwendung von sulfatierten Hyaluronsäuren, insbesondere in Form isotonischer und steriler Formulierungen, zur Herstellung eines Arzneimittels für die intraarticuläre Anwendung und Anwendungen im Sehnenbereich. Solche Formulierungen werden mit einem überraschenden Heilungserfolg zur Behandlung von entzündlichen und degenerativen Gelenk- und Sehnenerkrankungen im initialen, akuten und chronischen Stadium angewendet. Sie können erfindungsgemäß als höher konzentriertes Spritzpräparat appliziert werden und im Körper verbleiben oder als weniger konzentrierte Spülflüssigkeit eingesetzt werden. Es wurde bei den der Erfindung zu Grunde liegenden Untersuchungen eine sehr deutlich bessere Wirkung bei der Behandlung von Rheumatoid-Arthritis im Vergleich zu einer an sich bekannten Injektion oder Spülung mit isotonischen Hyaluronsäurelösungen gefunden.

Die erfindungsgemäße Verwendung der sulfatierten Hyaluronsäuren erfolgt in wässriger Lösung, insbesondere in isotonischer wässriger Lösung, welche therapeutisch wirksame Dosen von sulfatierter Hyaluronsäure mit applikationsspezifischem Sulfatierungsgrad enthalten. Solche Lösungen werden in den intraarticulären Spalt eines Gelenks oder in die Sehnenscheide oder in die Umgebung von Sehnen injiziert und verbleiben dort. Bei der Anwendung als Spülflüssigkeit wird mit den Formulierungen der intraarticuläre Spalt oder die Umgebung einer Sehne gespült und der Wirkstoff verbleibt nicht am Ort der Entzündung. In einer anderen Ausführung werden die sulfatierte Hyaluronsäuren enthaltende, höher viskosen Formulierungen ebenfalls als Injektionspräparat für den intraartikulären Spalt angewendet. In anderen erfindungsgemäßen Ausführungen werden hochviskose Lösungen injiziert oder die erfindungsgemäßen Gele oder Pasten werden, gegebenfalls unter erhöhter Druckanwendung in der Injektionsvorrichtung, über eine Kanüle oder Injektionsnadel in die Gelenkkapsel oder in die Umgebung der Sehne appliziert. Vorteilhaft ist die Anwendung der hochviskosen bzw. gelförmigen oder pastösen Formulierungen immer dann, wenn eine Depotbildung erwünscht ist.

Die in der erfindungsgemäßen Verwendung zur Anwendung gelangenden hochviskosen bis pastösen Formulierungen enthalten in einer weiteren Ausführungsform der Erfindung neben der sulfatierten Hyaluronsäure ein die Viskosität erhöhendes oder gelbildendes Hydrokolloid, bevorzugt Hyaluronsäure, als Hilfsstoff. Die Hyaluronsäure oder andere Hydrokolloide bewirken in der Regel eine höhere Viskosität bzw. eine Gel-Struktur der Formulierungen. Vorteilhaft bei Einsatz von Hyaluronsäure in den Formulierungen ist, dass die Hyaluronsäure zusätzlich einen chondroprotektiven Effekt bewirkt. Außer der Hyaluronsäure können beispielsweise andere polyanionische Polysaccharide wie Xanthan, Alginsäure oder Pectinsäure eingesetzt werden.

Anstelle der Hyaluronsäure wird in einer weiteren Ausführung auch das Uronid der Hyaluronsäure mit Vorteil eingesetzt. Das Uronid wird durch die enzymatische Spaltung der Hyaluronsäure mit dem mikrobiellen Enzym Hyaluronatlyase in an sich bekannter Weise hergestellt. Das Uronid enthält ungesättigte Bindungen an den endständigen Glucuronsäureresten. Das Uronid trägt auf Grund der im allgemeinen niedrigeren Molmassen weniger zur Erhöhung der Viskosität bei. Vorteilhaft ist die besonders hohe radikalbindende Eigenschaft, die über die Wirkung der Hyaluronsäure hinaus geht. Die entzündlichen Prozesse in den Gelenken oder der Umgebung der Sehnen werden noch intensiver abgeschwächt und die heilende Wirkung der Formulierungen verstärkt.

Die hochviskosen, pastösen bzw. gelförmigen Formulierungen werden beispielsweise auf folgendem Weg hergestellt. Einer sterilfiltrierte Flüssigformulierung, die gegebenfalls ein weiteres Hydrokolloid, z. B. Hyaluronsäure oder Hyaluronsäure-Uronid enthält, wird das Wasser, beispielsweise durch Gefriertrocknung unter Sterilbedingungen entzogen. Anschließend erfolgt die Zugabe von isotonisch eingestelltem sterilen Wasser in einer Menge, die zu einer hochviskosen bis pastösen Formulierung führt.

Die Injektionslösungen enthalten zwischen 1,0 mg/ml und 200,0 mg/ml, bevorzugt zwischen 10,0 mg/ml und 50,0 mg/ml sulfatierte Hyaluronsäure.

In den erfindungsgemäß angewandten Spüllösungen liegt die Konzentration der sulfatierten Hyaluronsäure zwischen 0,01 mg/ml und 20 mg/ml. Der Sulfatierungsgrad der Hyaluronsäure liegt in einer Ausführung, bevorzugt bei Anwendungen bei weniger massiv ausgeprägter Rheumatoid-Arthritis entweder im Bereich von 0,1 bis 2,0, bezogen auf eine Disaccharideinheit, deren maximaler Sulfatierungsgrad 4,0 betragen kann. In einer anderen Ausführung, die bevorzugt bei massiven Krankheitsbildern der Rheumatoid-Arthritis angewendet wird, liegt der Sulfatierungsgrad der erfindungsgemäß eingesetzten sulfatierten Hyaluronsäure im Bereich von 2,0 bis 4,0.

Die Molmassen der erfindungsgemäßen sulfatierten Hyaluronsäuren liegen zwischen 1.000 und 500.000 D. Die Isotonie der wässrigen Formulierungen wird durch einen Gehalt an anorganischen Salzen, bevorzugt Kochsalz, hervorgerufen.

Zur Unterstützung des Heilungseffektes bei der erfindungsgemäßen Verwendung von sulfatierten Hyaluronsäuren kann der Formulierung gegebenenfalls ein weiterer Wirkstoff, wie ein Antibiotikum oder eine zusätzliche entzündungshemmende Substanz, z.B. ein Cyclooxygenase-Inhibitor, zugesetzt werden.

Ohne die Erfindung dadurch einzuschränken, seien einige typische Anwendungsgebiete der Erfindung beschrieben, bei denen eine überraschende Hemmung von entzündlichen Arthritiden nachgewiesen wurde.
Als erfindungsgemäß angewandtes Injektionspräparat wird die Formulierung bei Erkrankungen des rheumatischen Formenkreises intraartikulär in den Gelenkspalt, in die kleinen Wirbelgelenke und die Hiosakralgelenke injiziert. Bei Tenosynovialitiden rheumatischer und idiopatischer Genese erwies sich die Injektion direkt in Sehnenscheiden oder in die weitere Umgebung der Sehnen als erfolgreich. Intraartikuläre Injektionen können auch nach arthroskopischen Eingriffen an allen großen und kleinen Gelenken, bei denen eine entzündliche Komponente der Gelenkschleimhaut ersichtlich ist, angewendet werden.
Es erwies sich auch als günstig für die Behandlung, wenn die Formulierung bei Arthrosen der kleinen und großen Gelenke (z.B. Coxarthrosen, Gonarthrosen, Omarthrosen) im entzündlichem Stadium eingesetzt wird.
In einer anderen Ausführung werden erfindungsgemäße Spüllösungen bevorzugt zu postoperativen Spülungen von arthroskopisch, endoprothetisch oder offen mittels Synovektomie versorgten großen und kleinen Gelenken, bei denen eine entzündliche Komponente der Gelenkschleimhaut ersichtlich ist (z.B. Rheumatoidarthritis, reaktive Arthritis oder aktivierte Arthrose), eingesetzt.

Durch den unterschiedlichen Sulfatierungrad der erfindungsgemäß eingesetzten sulfatierten Hyaluronsäure liegen Formulierungen vor, die an unterschiedliche Behandlungenarten angepaßt sind. Je höher der Sulfatierungsgrad ist, um so intensiver ist die spezifische Wirkung der sulfatierten Hyaluronsäure, während sich die chondroprotektiven Eigenschaften bzw. die Hyaluronsäure-spezifischen Eigenschaften verringern. Die Behandlungsintensität kann entsprechend des Krankheitsbildes variiert werden. Für Spülungen kann der Wirkstoff mit kleinerem Sulfatierungsgrad eingesetzt werden, ebenso in leichteren Erkrankungen oder auch im Vorfeld der Erkrankung. Bei schweren Entzündungsfällen wird hochsulfatierte Hyaluronsäure bevorzugt eingesetzt, später mit Abklingen der Erscheinungen auch Formulierungen, die weniger sulfatierte Hyaluronsäure und gegebenenfalls Hyaluronsäure enthalten.

Neben der unerwarteten, sehr intensiven Wirkung und der sehr guten Verträglichkeit auch hoher Dosen an sulfatierter Hyaluronsäure in den Formulierungen sind auch andere Eigenschaften der sulfatierten Hyaluronsäure im Vergleich zu Lösungen reiner Hyaluronsäure von Vorteil.
So ist vorteilhaft, dass die Lösungen der sulfatierten Hyaluronsäure im allgemeinen eine niedrigere Molmasse und demzufolge eine niedrigere Viskosität als gleichkonzentrierte Lösungen reiner Hyaluronsäure besitzen. Die sulfatierte Hyaluronsäure kann deshalb in höheren Konzentrationen und/oder in kleineren Volumina injiziert werden. Auch kann die Formulierung in einer sterilfiltrierten Form eingesetzt werden.

Im Vergleich zu den anderen Wirkstoffen ist sulfatierte Hyaluronsäure ein Abkömmling der humanidentischen Hyaluronsäure. Es kann vermutet werden, dass die nachgewiesene gute Verträglichkeit des Wirkstoffs mit der strukturellen Nähe des erfindungsgemäßen Wirkstoffes zu der nativen Hyaluronsäure bzw. zu den sulfatierten Glycosaminoglycanen in Zusammenhang steht. Ein weiterer großer Vorteil entsteht auch durch die thrombolytischen Eigenschaften der sulfatierten Hyaluronsäure. Dadurch wird gleichzeitig der Bildung von Thromben nach Verletzung von Blutgefäßen im und am Gelenk durch die Injektion vorgebeugt.

Die entzündungshemmende Wirkung im Gelenk- oder Sehnenbereich erscheint als eine spezifische Wirkung der sulfatierten Hyaluronsäure. Diese überraschende Wirkung, die über eine allgemeine Entzündungshemmung hinausgeht, war für die Anwendungen der erfindungsgemäßen Formulierungen im Gelenk und Sehnenbereich weder vorherzusagen noch vorher bekannt.

Die große Kapazität der erfindungsgemäß verwendeten Formulierungen, enthaltend sulfatierte Hyaluronsäure, zur Herstellung eines Arzneimittels zur spezifischen Behandlung der Arthritiden als auch beim Schutz des Knorpels im Vergleich zu unsulfatierter Hyaluronsäure wird durch eine experimentelle Untersuchung am Tier (Ausführungsbeispiel 2) in vivo nachgewiesen.

Die Bedeutung der Erfindung liegt in der Verwendung der sulfatierten Hyaluronsäure bei der Herstellung eines Arzneimittels zur Behandlung von Arthritiden sowie in deren Einfluß nicht nur auf den symptomatischen Befund der Abschwellung der Gelenke, sondern auch die Hemmung der Knorpel- und Knochendestruktion in allen Stadien der Erkrankung. Die Anwendung der beschriebenen Hyaluronsäurepräparate beschränkt sich bislang auf die Behandlung von Patienten mit Arthrose, die langsam degenerativ ist und einen vorwiegend nichtentzündlichen Verlauf hat. Der Einsatz der erfindungsgemäßen Formulierungen ist deshalb speziell für die sehr große Zahl von Patienten mit Rheumatoid-Arthritis von großer Bedeutung.

### Ausführungsbeispiel 1

### Bereitung der sterilen, pyrogenfreien Spritzlösung:

Es wurde eine pyrogenfreie sterilfiltrierte Hyaluronsäure aus *Streptococcus equisimilis* mit einer definierten Molmasse von 100 000 bis 3 000 000 Dalton verwendet. Die Molmasse wurde mit size exclusion chromatography (SEC)- multi angle laser light spektrometrie (MALLS) bestimmt. Sulfatierte Hyaluronsäure wurde durch Sulfatierung hochmolekularer Hyaluronsäure entsprechend
DE 19.813.234 A 1 erhalten und die Molmasse mit einer Lichtstreumethode (SEC-MALLS) bestimmt.

Eine 1%ige Hyaluronsäurelösung wird gegen pyrogenfreies Wasser solange dialysiert bis die Leitfähigkeit des Wassers unter 20 mS gesunken ist. Die Lösung wird lyophilisiert. 2 Liter einer 0,2%ige Hyaluronsäure werden mit Aktivkohle behandelt und über eine 1 bis 2 cm dicke Schicht Kieselgel des Typs "Köstrosorb" (Chemiewerk Bad Köstritz GmbH, Bad Köstritz) als Filterhilfsmittel filtriert. Danach wird über einen 0,8 µm und anschließend über einen 0.2 µm-Zelluloseacetat-Filter filtriert.
Die nun pyrogenfreie Hyaluronsäure wird unter sterilen Bedingungen lyophilisiert und danach mit steriler physiologischer Kochsalzlösung versetzt.

Für Vergleichszwecke wird eine 1%ige Lösung einer hochmolekularen Hyaluronsäure oder deren Salze in physiologischer NaCl-Lösung hergestellt und bei Raumtemperatur sterilfiltriert.

### Ausführungsbeispiel 2

### Prüfung der anti-inflammatorischen Wirkung am Kniegelenk der Ratte:

Bei dem verwendeten Tiermodell, der antigeninduzierten Arthritis der Ratte, handelt es sich um ein anerkanntes Tiermodell, welches die Mechanismen der Rheumatoid-Arthritis (RA) sehr gut widerspiegelt. Die histomorphometrischen Untersuchungen am unbehandelten Kniegelenk am Übergang zwischen Knorpel und Knochen ergaben typische Veränderungen, die in dieser Form bei Mäusen aufgrund der relativ starken Ausbildung der subchondralen Lamelle nicht gefunden werden.

Für den Versuch werden weibliche Wistar-Ratten eingesetzt. Im Abstand von einer Woche werden die Tiere subkutan mit 0,1 mg Methyl-Albumin (mBSA) in komplettem Freund'schen Adjuvans vorimmunisiert. Zwei Wochen nach der zweiten Immunisierung werden 0,1 mg mBSA intraarticulär in das linke Kniegelenk injiziert. Diese Injektion löst eine Arthritis aus, welche nach einer akuten Phase über Wochen persistiert. Einen Tag nach Auslösung der Arthritis erfolgt der Beginn der Behandlung mit sulfatierter Hyaluronsäurelösung oder mit hochmolekularer Hyaluronsäure (Mw = 1.800 kD), die i. art. in das Kniegelenk injiziert werden.

Untersucht werden Parameter, welche den Einfluß mikrobiell gewonnener hochmolekularer Hyaluronsäure und sulfatierter Hyaluronsäure auf die antigeninduzierte Arthritis beschreiben:
1. Lokalbefund der Arthritis (im Verlauf des Versuches)
2. Histologischer Arthritis-Score
3. Ausbildung und Morphologie von Mikrofrakturen im Bereich des Kalkknorpels
4. Messung des lateralen Gelenkdurchmessers
5. Biochemische Parameter

Der Versuchsablauf erfolgte nach Tabelle I

**Tabelle 1**

| Versuchsablauf | -3 Wo. | -2 Wo. | -1 Wo. | 0 | +1 Tag | +1 Wo. | +2 Wo. | +3 Wo. |
|---|---|---|---|---|---|---|---|---|
| Immunisierung 1 | V 1/2, | | | | | | | |
| | K1/2 | | | | | | | |
| Immunisierung 2 | | V.1/2, | | | | | | |
| | | K 1/2 | | | | | | |
| | | | | | | | | |
| Auslösung der Arthritis | | | | V 1/2, | | | | |
| | | | | K 1/2 | | | | |
| Sulfatierte Hyaluronsäure | | | | | V ½ | V 2 | V2 | V2 |
| Tötung | | | | | | V 1, K 1 | | V 2, K 2 |

| | | | | | | | | |
|---|---|---|---|---|---|---|---|---|
| 1=Kurzzeit 2=Langzeit V=Versuchsgr. (je 20 Tiere) K=Kontrollgr. (je 20 Tiere) | | | | | | | | |

Die Tiere wurden bei jedem Eingriff mittels Äthernarkose betäubt.

Die Immunisierung der Tiere erfolgte im Abstand von 7 Tagen. Dabei wurden bei der ersten Immunisierung insgesamt vier Injektionen von je 0,25 ml subcutan (entsprechend 0,1mg Methyl-Albumin in komplettem Freund'schen Adjuvans) beidseits der Wirbelsäule in Höhe der Schulterblätter und oberhalb der Hüfte (je etwa 1,5 cm Abstand von den Dornfortsätzen) durchgeführt. Bei der 2. Immunisierung wurden 3 Depots mit je 0,33 ml Lösung gesetzt; einmal genau zwischen beide Schulterblättern und 2x in Höhe des Abdomens beidseits paravertebral.

Die Auslösung der Arthritis erfolgte 2 Wochen nach der 2. Immunisierung durch i.a. Injektion von jeweils 0,05 ml einer 10 mg/ml Lösung mBSA in physiologischer Kochsalzlösung ins jeweils rechte Kniegelenk.
Am Tag nach Auslösung der Arthritis wurde den 20 Tieren der Versuchsgruppe jeweils 0,05 ml eine Formulierung, enthaltend sulfatierte HA ins rechte Kniegelenk (Verwendung einer Insulinspritze, dünne Kanüle 0,35x40mm) gespritzt. Danach erfolgte wöchentlich einmal die Injektion (Langzeitgruppe)
Es wurde der laterale Gelenkdurchmesser mittels Abstandsmesser (Fa. Matatuyo) aller Tiere vor, 1, 4 und 8 Tage nach der Auslösung der Arthritis und dann jede Woche bis zum Versuchsabschluss gemessen. Die Tiere wurden parallel dazu gewogen.
Alle Tiere wiesen bereits am folgenden Tag nach Auslösen der Arthritis eine Schwellung des rechten Kniegelenks auf. Das Gewicht wies keine starken Schwankungen auf.
Am 7. Tag wurden 10 Tiere der Versuchsgruppe und 10 Tiere der Kontrollgruppe getötet. Die Tiere wurden mittels Äthernarkose eingeschläfert, die Tötung der restlichen Tiere erfolgte 1 Woche nach der letzten Injektion einer Formulierung, enthaltend sulfatierter HA, ins rechte Kniegelenk.

Insbesondere der Langzeitversuch über 3 Wochen zeigte, dass eine signifikante Abnahme der Gelenkdurchmesser (als Maß für die entzündliche Schwellung) bei den mit sulfatierter HA behandelten Tieren nachweisbar ist.

### Tabelle 2 Gelenkdurchmesser (rechts)

Für den 0. Tag wird der Gelenkdurchmesser angegeben.
Vom 0. zum 1, Tag wird der Anstieg des Gelenkdurchmessers durch die Immunisierung angegeben vom 2 bis 29. Tag wird der Abfall bzw. Anstieg des Gelenkdurchmessers nach Spritzung am 1. Tag angegeben.

### Kurzzeitversuch

| | 0Tag | 1 Tag | 4 Tage | 8 Tage |
|---|---|---|---|---|
| Sulfatierte HA | 867,4 | +228 | +20 | -118 |
| HA | 913,2 | +206 | +18 | -45 |

### Langzeitversuch

| | 0 Tag | 1 Tag | 4 Tage | 8 Tage | 15 Tage | 22 Tage | 29 Tage |
|---|---|---|---|---|---|---|---|
| Sulfatierte | 927,6 | 1153,9 | | | | | |
| HA | | +226 | -4 | -127 | -203 | -212 | -231 |
| HA | 926,0 | 1132,9 | | | | | |
| | | +206 | +27 | -97 | -140 | -144 | -134 |
| Kontrolle | 867,4 | 1095,3 | | | | | |
| | | +228 | +20 | -45 | -100 | -190 | -174 |

| | | | | | | | |
|---|---|---|---|---|---|---|---|
| Meßwert am 1 Tag Differenz zu 0 Tag | | | | | | | |

### Ausführungsbeispiel 3

Herstellung einer Spülflüssigkeit: Es werden 1,0 g sulfatierte Hyaluronsäure mit einer Molmasse von 150.000 D und 1,0 g Hyaluronsäure-Uronid mit einer durchschnittlichen Molmasse von 20.000 D in einem Liter physiologischer NaCl-Lösung gelöst und die Lösung bei Raumtemperatur sterilfiltriert.

## Patentansprüche

1. Verwendung von sulfatierten Hyaluronsäuren zur Herstellung eines Arzneimittels zur Behandlung von entzündlicher Arthritiden bei Mensch und Tier, wobei die sulfatierten Hyaluronsäuren wasserlöslich sind.

2. Verwendung gemäß Anspruch 1, **dadurch gekennzeichnet, dass** eine Formulierung ausgewählt aus isotonischen wässrigen bis hochviskosen Lösungen, Gelen und Pasten verwendet wird.

3. Verwendung gemäß Anspruch 2, **dadurch gekennzeichnet, dass** die Formulierung einen weiteren Wirkstoff und/oder einen oder mehrere Hilfsstoffe enthält.

4. Verwendung gemäß Anspruch 2, **dadurch gekennzeichnet, dass** die Konzentration der sulfatierten Hyaluronsäure in flüssigen Formulierungen zwischen 1,0 mg/ml und 200,0 mg/ml, bevorzugt zwischen 10,0 mg/ml und 50,0 mg/ml, liegt.

5. Verwendung gemäß Anspruch 2, **dadurch gekennzeichnet, dass** die Konzentration der sulfatierten Hyaluronsäure in einer Flüssigkeit bei Applikation in den intraartikulären Spalt oder in die Umgebung einer Sehne zwischen 0,01 mg/ml und 20 mg/ml liegt.

6. Verwendung gemäß Anspruch 2, **dadurch gekennzeichnet, dass** der Sulfatierungsgrad der Hyaluronsäure in einer Injektionslösung im Bereich von 0,1 bis 2 liegt.

7. Verwendung gemäß Anspruch 2, **dadurch gekennzeichnet, dass** der Sulfatierungsgrad der Hyaluronsäure in einer Spülflüssigkeit im Bereich von 2 bis 4 liegt.

8. Verwendung gemäß Anspruch 1, **dadurch gekennzeichnet, dass** die Molmassen der sulfatierten Hyaluronsäure zwischen 1.000 und 500.000 liegen.

9. Verwendung gemäß Anspruch 3, **dadurch gekennzeichnet, dass** die Formulierungen zusätzlich ein Hydrokolloid enthalten

10. Verwendung gemäß Anspruch 9, **dadurch gekennzeichnet, dass** die Formulierungen Hyaluronsäure enthalten.

11. Verwendung gemäß Anspruch 3, **dadurch gekennzeichnet, dass** die Formulierungen zusätzlich ein Uronid der Hyaluronsäure enthalten.

12. Verwendung gemäß Anspruch 2, **dadurch gekennzeichnet, dass** die Formulierungen für intraartikuläre Injektionen in die kleinen Wirbelgelenke und die Iliosakralgelenke bei Erkrankungen des rheumatischen Formenkreises geeignet sind.

13. Verwendung gemäß Anspruch 2, **dadurch gekennzeichnet, dass** die Formulierungen für intraartikuläre Injektionen in die Umgebung der Sehnen bei Tenosynovialitiden rheumatischer und idiopatischer Genese geeignet sind.

14. Verwendung gemäß Anspruch 2, **dadurch gekennzeichnet, dass** die Formulierung für intraartikuläre Injektionen nach arthroskopischen Eingriffen an großen und kleinen Gelenken, bei denen eine entzündliche Komponente der Gelenkschleimhaut ersichtlich ist, geeignet sind

15. Verwendung gemäß Anspruch 2, **dadurch gekennzeichnet, dass** die Formulierung für intraartikuläre Injektionen bei Arthrosen der kleinen und großen Gelenken wie Coxarthrosen, Gonarthrosen oder Omarthrosen im entzündlichen Stadium geeignet sind

16. Verwendung gemäß Anspruch 2, **dadurch gekennzeichnet, dass** die Formulierungen als Spülung und/oder als Injektion sowohl im initialen als auch im akuten oder chronischen Stadium bei Erkrankungen des rheumatischen Formenkreises geeignet sind.

17. Verwendung gemäß Anspruch 2, **dadurch gekennzeichnet, dass** die Formulierung für postoperative Spülungen von arthroskopisch, endoprothetisch oder offen mittels Synovektomie versorgten großen und kleinen Gelenken, bei denen eine entzündliche Komponente der Gelenkschleimhaut ersichtlich ist, wie bei rheumatoider Arthritis, reaktive Arthritis oder aktivierte Arthrose geeignet ist

18. Verwendung von sulfatierten Hyaluronsäuren gemäß Anspruch 2, **dadurch gekennzeichnet, dass** die Formulierung für Lokalinjektionen nach Bandscheibenoperationen zur Prophylaxe entzündlicher Veränderungen und daraus folgender narbiger Verklebungen im Sinne eines Postnucleotomiesyndroms geeignet ist.

## Claims

1. Use of sulfated hyaluronic acids for manufacturing a pharmaceutical agent for the treatment of inflammatory arthritides in humans and animals, wherein the sulfated hyaluronic acids are water-soluble.

2. The use according to claim 1, **characterized in that** a formulation selected from isotonic aqueous to highly viscous solutions, gels and pastes is used.

3. The use according to claim 2, **characterized in that** the formulation contains an additional active ingredient and/or one or several adjuvants.

4. The use according to claim 2, **characterized in that** the concentration of the sulfated hyaluronic acid in liquid formulations is from 1.0 mg/ml to 200.0 mg/ml, preferably from 10.0 mg/ml to 50.0 mg/ml.

5. The use according to claim 2, **characterized in that** the concentration of the sulfated hyaluronic acid in a liquid is from 0.01 mg/ml to 20 mg/ml during an administration into an intraarticular gap or in the vicinity of a tendon.

6. The use according to claim 2, **characterized in that** the degree of sulfation of the hyaluronic acid in an injection solution is in the range from 0.1 to 2.

7. The use according to claim 2, **characterized in that** the degree of sulfation of the hyaluronic acid in a rinsing liquid is in the range from 2 to 4.

8. The use according to claim 1, **characterized in that** the molecular weights of the sulfated hyaluronic acids are from 1000 to 500,000.

9. The use according to claim 3, **characterized in that** the formulations additionally contain a hydrocolloid.

10. The use according to claim 9, **characterized in that** the formulations contain hyaluronic acid.

11. The use according to claim 3, **characterized in that** the formulations additionally contain a uronide of hyaluronic acid.

12. The use according to claim 2, **characterized in that** the formulations for intraarticular injections into the small vertebral joints and the sacroiliac joints are suited for diseases of the rheumatic sphere.

13. The use according to claim 2, **characterized in that** the formulations are suited for intraarticular injections in the vicinity of tendons in tenosynovitides of rheumatic and idiopathic genesis.

14. The use according to claim 2, **characterized in that** the formulation is suited for intraarticular injections after arthroscopic interventions in large and small joints where an inflammatory component of the articular synovium is apparent.

15. The use according to claim 2, **characterized in that** the formulation is suited for intraarticular injections with arthroses of small and large joints such as coxarthroses, gonarthroses or omarthroses in the inflammatory stage.

16. The use according to claim 2, **characterized in that** the formulations are suited as an irrigation fluid and/or injection both in the initial and the acute or chronic stages of diseases of the rheumatic sphere.

17. The use according to claim 2, **characterized in that** the formulation is suited for postoperative irrigation fluids for the arthroscopic, endoprothetic treatment or open treatment by synovectomy of large and small joints where an inflammatory component of the articular synovium is apparent, such as rheumatoid arthritis, reactive arthritis or activated arthritis.

18. The use of sulfated hyaluronic acids according to claim 2, **characterized in that** the formulation is suited for local injections subsequent to intervertebral disk operations for the prophylaxis of inflammatory changes and cicatrized adhesions resulting therefrom within the meaning of a postnucleotomy syndrome.

## Revendications

1. Utilisation d'acides hyaluroniques sulfatés pour la fabrication d'un médicament destiné au traitement d'arthrites inflammatoires chez l'Homme et l'animal, dans laquelle les acides hyaluroniques sulfatés sont hydrosolubles.

2. Utilisation selon la revendication 1, **caractérisée en ce que** l'on utilise une formulation choisie parmi les solutions isotoniques aqueuses à très visqueuses, les gels et les pâtes.

3. Utilisation selon la revendication 2, **caractérisée en ce que** la formulation contient un autre principe actif et/ou un ou plusieurs adjuvants.

4. Utilisation selon la revendication 2, **caractérisée en ce que** la concentration de l'acide hyaluronique sulfaté dans les formulations liquides est comprise entre 1,0 mg/mL et 200,0 mg/mL, de préférence entre 10,0 mg/mL et 50,0 mg/mL.

5. Utilisation selon la revendication 2, **caractérisée en ce que**, lors d'une administration dans l'espace intra-articulaire ou au voisinage d'un tendon, la concentration de l'acide hyaluronique sulfaté dans un liquide est comprise entre 0,01 mg/mL et 20 mg/mL.

6. Utilisation selon la revendication 2, **caractérisée en ce que** le degré de sulfatation de l'acide hyaluronique dans une solution pour préparation injectable est dans la gamme de 0,1 à 2.

7. Utilisation selon la revendication 2, **caractérisée en ce que** le degré de sulfatation de l'acide hyaluronique dans un liquide de lavage est dans la gamme de 2 à 4.

8. Utilisation selon la revendication 1, **caractérisée en ce que** les masses moléculaires de l'acide hyaluronique sulfaté sont comprises entre 1 000 et 500 000.

9. Utilisation selon la revendication 3, **caractérisée en ce que** les formulations contiennent en plus un hydrocolloïde.

10. Utilisation selon la revendication 9, **caractérisée en ce que** les formulations contiennent de l'acide hyaluronique.

11. Utilisation selon la revendication 3, **caractérisée en ce que** les formulations contiennent en plus un uronide de l'acide hyaluronique.

12. Utilisation selon la revendication 2, **caractérisée en ce que** les formulations sont appropriées pour des injections intra-articulaires dans les petites articulations vertébrales et les articulations sacro-iliaques dans le cas de maladies de type rhumatismal.

13. Utilisation selon la revendication 2, **caractérisée en ce que** les formulations sont appropriées pour des injections intra-articulaires au voisinage des tendons dans le cas de ténosynovites d'origine rhumatismale ou idiopathique.

14. Utilisation selon la revendication 2, **caractérisée en ce que** la formulation est appropriée pour des injections intra-articulaires à la suite d'interventions arthroscopiques sur grandes et petites articulations dans lesquelles un état inflammatoire de la membrane synoviale est détecté.

15. Utilisation selon la revendication 2, **caractérisée en ce que** la formulation est appropriée pour des injections intra-articulaires dans le cas d'arthroses de petites et grandes articulations telles que les coxarthroses, les gonarthroses ou les omarthroses au stade inflammatoire.

16. Utilisation selon la revendication 2, **caractérisée en ce que** les formulations sont appropriées en tant que lavage et/ou injection, tant au stade initial qu'au stade aigu ou chronique de maladies de type rhumatismal.

17. Utilisation selon la revendication 2, **caractérisée en ce que** la formulation est appropriée pour des lavages post-opératoires de grandes et petites articulations traitées par arthroscopie, endoprothèse ou à ciel ouvert par synovectomie, dans lesquelles un état inflammatoire de la membrane synoviale est détecté comme dans le cas de la polyarthrite rhumatoïde, de l'arthrite réactionnelle ou de l'arthrose activée.

18. Utilisation d'acides hyaluroniques sulfatés selon la revendication 2, **caractérisée en ce que** la formulation est appropriée pour des injections locales à la suite d'opérations du disque intervertébral en vue de la prophylaxie de modifications inflammatoires et d'adhérences cicatricielles consécutives dues à un syndrome de post-nucléotomie.
